(19) Europäisches Patentamt
European Patent Office
Office européen des brevets

(11) EP 2 654 678 B1

# (12) EUROPEAN PATENT SPECIFICATION

(45) Date of publication and mention of the grant of the patent:
**15.04.2015 Bulletin 2015/16**

(21) Application number: **11799273.5**

(22) Date of filing: **12.12.2011**

(51) Int Cl.:
***A61K 8/22*** (2006.01) ***A61K 8/49*** (2006.01)
***A61K 8/66*** (2006.01) ***A61Q 5/10*** (2006.01)

(86) International application number:
**PCT/EP2011/072433**

(87) International publication number:
**WO 2012/084568 (28.06.2012 Gazette 2012/26)**

## (54) A METHOD OF COLOURING HAIR FIBRES

VERFAHREN ZUM FÄRBEN VON HAARFASERN

PROCÉDÉ DE COLORATION DE FIBRES CAPILLAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2010 EP 10195893**

(43) Date of publication of application:
**30.10.2013 Bulletin 2013/44**

(73) Proprietors:
- **Unilever PLC, a company registered in England and**
  **Wales under company no. 41424**
  **Greater London EC4Y 0DY (GB)**
  Designated Contracting States:
  **CY GB IE MT**
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
- **BHOGAL, Ranjit,Kaur**
  **Bedford Bedfordshire MK44 1LQ (GB)**
- **CASEY, John**
  **Bedford Bedfordshire MK44 1LQ (GB)**
- **HUNTER, Karl, John**
  **Bedford Bedfordshire MK44 1LQ (GB)**
- **GANGULI, Shovan**
  **Bangalore 560 066 (IN)**
- **KOEK, Jean, Hypolites**
  **NL-3133 AT Vlaardingen (NL)**
- **REDFERN, Sally,Pamela**
  **Bedford Bedfordshire MK44 1LQ (GB)**

(74) Representative: **James, Helen Sarah**
**Unilever PLC**
**Colworth House**
**Sharnbrook**
**Bedford**
**MK44 1LQ (GB)**

(56) References cited:
**US-A- 3 957 424**

- **TAKADA ET AL.: JOURNAL OF OLEO SCIENCE, vol. 52, no. 10, 20 March 2001 (2001-03-20), pages 557-563, XP002637133, cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 654 678 B1

## Description

A method of colouring hair fibres is disclosed, the method comprising the step of applying a hair colour composition based on (+)-catechin, (-)-catechin, (+)-epicatechin, (-)-epicatechin or mixtures thereof and an enzyme, in particular a peroxidase. The hair colour composition also includes a hydrogen peroxide generator or hydrogen peroxide per se. Optionally the hair colour composition includes metal ions, for example iron or copper, which are able to generate a range of colours for dyeing hair through coordination chemistry with (+)-catechin, (-)-catechin, (+)-epicatechin, (-)-epicatechin or mixtures thereof, or reaction products thereof with the enzyme.

Hair colourant formulations fall into three main categories designated permanent, semi-permanent and temporary. They vary in their degree of wash-fastness where permanent hair colourant formulations last 30-40 shampoo cycles (4-6 weeks) and temporary systems are rinsed out during the first wash. Permanent hair colourant formulations are oxidative dye systems and generally contain paraphenylene diamine (PPD) and resorcinol, both of which have been shown to cause sensitisation and mutagenicity. Furthermore the severe oxidising conditions required (hydrogen peroxide concentrations of 3 % or more and a pH of 8.5 or higher for time periods of 20 minutes or more) in themselves can cause skin irritation and sensitization for some individuals as well as hair fibre damage. In addition, permanent hair colourant formulations also contain ammonia, used to swell the hair and lift the cuticle scales to allow penetration of dye precursors, which gives off a strong and unpleasant odour.

Takada et al. (Journal of Oleo Science, 52, 10, 557-563 (2003)) discloses a hair colouring composition comprising laccase and catechin at pH 5.

US 6 572 843 (Novozymes A/S) discloses a method for treating hair combining permanent dyeing and straightening of hair. After application of a relaxer to reduce the covalent disulphide linkages in the keratinous fibres of the hair thereby rendering the hair deformable, a Coprinus sp. peroxidase, hydrogen peroxide and a mediator solution consisting of paraphenylene diamine, 3-aminophenol and 4-aminophenol at pH 5.5 is applied to thereby colour the fibres and simultaneously neutralise the relaxer and fix the hair (by re-establishing the network of crosslinks in the keratinous fibres).

US 3 957 424 (The Proctor & Gamble Company) discloses an oxidative colouring process for hair based on hydrogen peroxide and soybean peroxidase which can be carried out under mild oxidising conditions (0.01-1.0 % by weight hydrogen peroxide and pH 4 to 10) not requiring a dual oxidation dye precursor comprising an aromatic polyhydric compound and an aromatic amine. In fact either can be used alone as the oxidation dye precursor. What is claimed is a process for colouring hair comprising contacting hair with an effective amount of a solution containing from about 0.01 to about 500 ppm (0.01 to 500 μg/mL) soybean peroxidase, from about 0.01 to about 1.0 % by weight hydrogen peroxide, and from about 0.001 to about 6 % by weight of an aromatic oxidation dye precursor, and having a pH of from about 4 to 10. In particular, a hair dyeing kit is disclosed comprising a 4 ounce bottle of 1 % by weight hydrogen peroxide, 2 g of carboxymethyl cellulose, 20 g starch, 0.07 g soybean peroxidase, 3 g pH 7 buffer and 4 g paraphenylene diamine or equivalent amounts of 4-hydroxy-3-methoxycinnamic acid (ferulic acid) or 2-methoxy-4-(1-propenyl)phenol (isoeugenol). Both latter compounds come under the broad term of phenylpropanoids as they are derived from phenylalanine and the phenylpropanoid pathway, ferulic acid being more specifically a phenylpropenoid and isoeugenol being more specifically a phenylpropene.

The inventive method addresses the aforementioned disadvantages by providing a method of colouring hair fibres which avoids the issues of safety and toxicity associated with the known synthetic oxidative systems, causes less damage by avoiding the use of hydrogen peroxide per se, whilst maintaining colour fastness.

Summary of the invention

In a first aspect of the invention, a method of colouring hair fibres is provided, the method comprising the step of applying a hair colour composition, the hair colour composition comprising:

(a) (+)-Catechin, (-)-catechin, (+)-epicatechin, (-)-epicatechin or mixtures thereof;
(b) A hydrogen peroxide generator or hydrogen peroxide; and
(c) A peroxidase and;

wherein the composition has a pH of 4.5 to 7.0, preferably less than or equal to 6.0.

As consumers will benefit from avoiding the issues around allergic skin reactions, safety and toxicity of current permanent hair colourant formulations, the hair colour composition may be applied more frequently, for example daily, in the form of, for example, a shampoo or conditioner thereby addressing the problem of grey hair or natural hair colour re-growth observed during the intervals between hair dyeing treatments. Furthermore by multiple consecutive applications of the hair colour composition, the consumer can also control the degree of colour desired.

The hair colour composition may comprise 0.01 - 10, preferably 0.1 - 5 % w/w (+)-catechin, (-)-catechin, (+)-epicatechin, (-)-epicatechin or mixtures thereof.

The peroxidase is selected from those which are suited to neutral or slightly acid pH, that is to say a pH of 4.5 to 7.0, preferably a pH of less than or equal to 6.0. The peroxidase is preferably a non-animal haem peroxidase from class II (fungi) or class III (plants and algae), most preferably obtained from the group consisting of Arabidopsis thaliana, horse radish, barley, peanut soybean, tobacco, and turnip (plants), Chlorophyta spirogyra (green algae), Arthromyces ramosus and Corprinus cinereus (fungi). The hair colour composition may comprise 0.0001 - 5, preferably 0.001 - 1 % w/w peroxidase.

A hydrogen peroxide generator is preferred because it reduces the aforementioned problems associated with the use of large amounts of hydrogen peroxide whilst providing a constant supply of hydrogen peroxide. Preferably the hydrogen peroxide generator comprises a hydrogen peroxide generating oxidase, a substrate and oxygen. Alternatively a chemical system can be used to generate hydrogen peroxide such as one based on ascorbate and a transition metal ion, or anthraquinone monosulphate and glucose (T. Vuorinen, Carbohydrate Research, 127, 2, 319-325 (15 April 1984)).

The hydrogen peroxide generating oxidase may be selected from the group consisting of (S)-2-hydroxy acid oxidase, D-galactose oxidase, glucose oxidase, coniferyl alcohol oxidase, glycolate oxidase, hexose oxidase, oxalate oxidase, amino acid oxidase and L-galactonolactone oxidase and the respective substrate is selected from the group consisting of (S)-2-hydroxy acid, D-galactose, glucose, coniferyl alcohol, $\alpha$-hydroxy acids, D-glucose, oxalic acid, amino acid and L-galactono-1,4-lactone.

More preferably the hydrogen peroxide generator is selected from the group consisting of (S)-2-hydroxy acid with (S)-2-hydroxy acid oxidase, D-galactose with D-galactose oxidase, glucose with glucose oxidase, coniferyl alcohol with coniferyl alcohol oxidase, $\alpha$-hydroxy acids with glycolate oxidase, D-glucose with hexose oxidase, oxalic acid with oxalate oxidase, amino acid oxidase with amino acid and L-galactono-1,4-lactone with L-galactonolactone oxidase, all in the presence of oxygen.

The hair colour composition may comprise 0.0001 - 3 preferably 0.001 - 1, most preferably 0.01 - 1 % w/w hydrogen peroxide. However if a hydrogen peroxide generator is selected, then the hair colour composition may comprise 0.0001 - 5, preferably 0.001 - 1 % w/w hydrogen peroxide generating oxidase, and 0.01 - 10, preferably 0.1 - 5 % w/w substrate.

The method of the first aspect may additionally comprise a metal ion suitable for coordinating to (+)-catechin, (-)-catechin, (+)-epicatechin, (-)-epicatechin or mixtures thereof, or the product of the reaction of (+)-catechin, (-)-catechin, (+)-epicatechin, (-)-epicatechin or mixtures thereof in the presence of the peroxidase and hydrogen peroxide. The metal ion may be selected from the group consisting of iron (II), iron (III), copper (I), copper (II), and aluminium (III). The hair colour composition may comprise 0.0001 - 2, preferably 0.001 - 0.1 % w/w metal ion.

Brief description of the figures

The invention will now be illustrated with reference to the figures which show in:

Figure 1 the $\Delta$E (figure 1a) and L* (figure 1b), a* (figure 1c) and b* (figure 1d) respectively for each of the reactions described in example 1 on the basis of six repeat measurements for each hair switch;

Figure 2 the $\Delta$E (figure 2a) and L* (figure 2b), a* (figure 2c) and b* (figure 2d) respectively for cumulative colour uptake described in example 2 on the basis of five repeat measurements for each hair switch; and

Figure 3 the $\Delta$E (figure 3a) and L* (figure 3b), a* (figure 3c) and b* (figure 3d) respectively for cumulative colour uptake described in example 3 on the basis of five repeat measurements for each hair switch wherein the $\Delta$E values take the x5 cumulative colour uptake as the baseline and "0" represents the L*, a* and b* values after x5 cumulative colour uptake.

**Detailed description of the invention**

**Example 1: A comparison between peroxidase and laccase**

Materials:

Myceliphthora thermophila laccase (51003) was obtained from Novozymes A/S (Denmark) Horse radish peroxidase (HRP) was obtained from Sigma (UK)
3 % aqueous hydrogen peroxide ($H_2O_2$) was obtained from Sigma (UK)
(+)-Catechin hydrate was obtained from Sigma (UK)
Natural White hair switches were obtained from International Hair Importers (New York, USA)

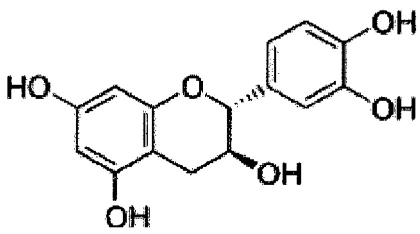

Catechin

Method:

Replicate baseline L*a*b* values of the hair switches (~0.5g) were determined using a Minolta model CM508d spectrophotometer. The hair switches were then incubated in reactions set up as follows (final concentrations in brackets) at 30 degrees centigrade for 15 minutes:

Laccase reaction:

| | |
|---|---|
| 300 µl | 100 mg/ml (+)-catechin hydrate (5 mg/ml) |
| 60 µl | 1000 U/ml laccase (10 U/ml) |
| 5640 µl | 50 mM succinate buffer pH 5.0 |

Horse radish peroxidase/hydrogen peroxide reaction:

| | |
|---|---|
| 300 µl | 100 mg/ml (+)-catechin hydrate (5 mg/ml) |
| 120 µl | 500 U/ml horse radish peroxidase (10 U/ml) |
| 600 µl | 3 % aqueous hydrogen peroxide (0.3 %) |
| 4980 µl | 50 mM succinate buffer pH 5.0 |

(+)-Catechin control

| | |
|---|---|
| 300 µl | 100 mg/ml (+)-catechin hydrate (5 mg/ml) |
| 5700 µl | 50 mM succinate buffer pH 5.0 |

No horse radish peroxidase control

| | |
|---|---|
| 300 µl | 100 mg/ml (+)-catechin hydrate (5 mg/ml) |
| 600 µl | 3 % aqueous hydrogen peroxide (0.3 %) |
| 5100 µl | 50 mM succinate buffer pH 5.0 |

No hydrogen peroxide control

| | |
|---|---|
| 300 µl | 100 mg/ml (+)-catechin hydrate (5 mg/ml) |
| 120 µl | 500 U/ml horse radish peroxidase (10 U/ml) |
| 5580 µl | 50mM succinate buffer pH 5.0 |

Buffer only control

6000 μl 50mM succinate buffer pH 5.0

The hair switches were then rinsed in distilled water, washed with shampoo, rinsed again as previously and dried using a hair dryer for approximately one minute. Colour generation following treatment was then evaluated using the Minolta model CM508d spectrophotometer. Replicate L*a*b* readings were determined and ΔE values calculated therefrom according to the equation below:

$$\Delta E = \sqrt{} (L*_B - L*_D)^2 + (a*_B - a*_D)^2 + (b*_B - b*_D)^2$$

Where B = background/baseline and D = dyed;
L* = lightness (where 0 = black and 100 = diffuse white);
a* = green/red (negative values indicate green and positive values indicate red); and
b* = blue/yellow (negative values indicate blue and positive values indicate yellow)

Results:

Figures 1a, 1b, 1c and 1d show respectively the results for the ΔE and L*, a* and b* for each of the abovementioned reactions on the basis of six repeat measurements at different regions of each hair switch

Conclusion:

Horse radish peroxidase in combination with hydrogen peroxide is more effective than laccase alone for generating colour on hair switches when (+)-catechin hydrate is used as a substrate. In particular, an increase in ΔE was observed using horse radish peroxidase and hydrogen peroxide compared to laccase. In terms of the L*a*b* colour space dimensions, a decrease in L* (lightness) and an increase (more red) in a* (green/red) and an increase (more yellow) in b* (blue/yellow) was observed when using horse radish peroxidase and hydrogen peroxide compared to laccase.

Example 2: A comparison of cumulative colour uptake over dyeing cycles, and colour stability after shampooing between (+)-catechin hydrate and three phenylpropenoids used for enzymatic hair colouring

Materials:

Caffeic acid (3,4-dihydroxycinnamic acid) was obtained from Sigma (UK)
Ferulic acid was obtained from Sigma (UK)
Isoeugenol was obtained from Sigma (UK)
Soybean peroxidase was obtained from Bio-Research Products Incorporated

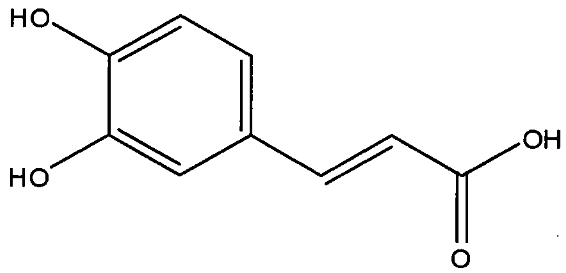

Caffeic acid

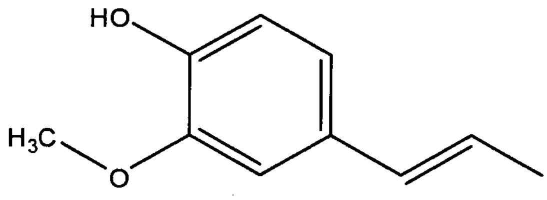

Isoeugenol

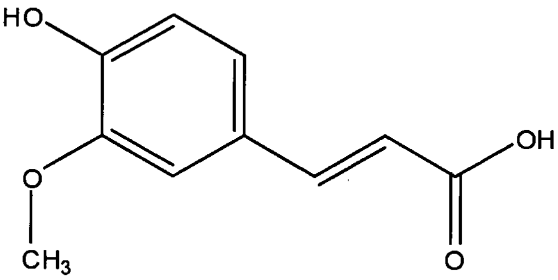

Ferulic acid

General method:

The general reaction conditions were (final concentrations in brackets):

Oxidation dye precursor (50 mM) (see table below for actual amounts used)
1 ml 3% hydrogen peroxide (0.3 %)
50 μl 1mg/ml or 1840U/mg soybean peroxidase (5 ppm or 9.4U/ml)
Balance to 10 ml of 100 mM $NaH_2PO4/Na_2HPO4$ buffer pH 6.2

which for each oxidation dye precursor meant:

| Oxidation dye precursor | % weight | Amount used per 10 ml (g) (adjusted for purity) |
|---|---|---|
| (+)-Catechin | 1.45 | 0.1481 |
| Caffeic acid | 0.90 | 0.0919 |
| Ferulic acid | 0.97 | 0.0981 |
| Isoeugenol | 0.82 | 0.0838 |

Cumulative colour uptake method:

One ~0.5g Natural White hair switches were allocated to each dye treatment and five replicate baseline L*a*b* colour measurements per switch taken using a Minolta model CM508d spectrophotometer.

Each of the oxidation dye precursors was weighed out into duplicate glass vials. 2 ml of ethanol (98%) was added to each vial except to isoeugenol where 3 ml was added to maintain solubility. 1 ml of 3 % aqueous hydrogen peroxide was then added to each vial and the pH 6.2 phosphate buffer added to make the total volume up to 9.950 ml in each vial.

A switch of hair was placed into each vial ensuring full coverage of the switch by agitation and incubated for 10 minutes. Thereafter 50 μl of a 1 mg/ml soybean peroxidase solution was added to each vial and the switch and dye solution again thoroughly agitated to ensure full coverage of the switch. The switches were incubated for a further 10 minutes and then rinsed in warm running tap water until the water ran clear. The switches were then combed and dried with a hair dryer. Colour measurements were then taken in quintuplicate per hair switch using the Minolta model CM508d spectrophotometer. The above process was repeated five times and cumulative colour build up measured per treatment.

The switches were then subjected to five cycles of washing with shampoo, drying and L*a*b* colour measurements using a Minolta model CM508d spectrophotometer. The switches were washed thoroughly for two minutes by adding 100 μl shampoo per switch, rubbed and combed in shampoo for one minute, and rinsed thoroughly in warm running water. The switches were then dried with a hair dryer.

Results:

Figures 2a, 2b, 2c and 2d show respectively the results for the ΔE and L*, a* and b* for cumulative colour

uptake on the basis of five repeat measurements for each hair switch. "0" represents the L*, a* and b* values for untreated natural white hair.

Figures 3a, 3b, 3c and 3d show respectively the results for the ΔE and L*, a* and b* for cumulative wash out on the basis of five repeat measurements for each hair switch wherein the ΔE values take the x5 cumulative colour uptake as the baseline and "0" represents the L*, a* and b* values after x5 cumulative colour uptake.

Conclusion:

(+)-Catechin hydrate (a flavonoid) shows after a single cycle of colour treatment the greatest colour shift to the red end of the spectrum (a* increases). After five cycles of colour treatment, the (+)-catechin hydrate still shows the greatest colour shift to the red end of the spectrum. This shift to the red end of the spectrum is particularly desired in a hair dye as a good red dye is considered a gold standard in the hair colour industry. Furthermore the shift to the red end of the colour spectrum with (+)-catechin hydrate appears to be colour fast as shown in figure 3c.

## Claims

1. A method of colouring hair fibres, the method comprising the step of applying a hair colour composition, the hair colour composition comprising:

   (a) (+)-Catechin, (-)-catechin, (+)-epicatechin, (-)-epicatechin or mixtures thereof;
   (b) A hydrogen peroxide generator or hydrogen peroxide; and
   (c) A peroxidase and;

   wherein the composition has a pH of 4.5 to 7.0, preferably less than or equal to 6.0.

2. A method of colouring hair fibres according to claim 1, wherein the peroxidase is a non-animal haem peroxidase from class II (fungi) or class III (plants and algae).

3. A method of colouring hair fibres according to claim 2, wherein the peroxidase is obtained from the group consisting of Arabidopsis thaliana, horse radish, barley, peanut soybean, tobacco, and turnip (plants), Chlorophyta spirogyra (green algae), Arthromyces ramosus and Corprinus cinereus (fungi).

4. A method of colouring hair fibres according to any one of the preceding claims, wherein the hair colour composition comprises 0.01 - 10, preferably 0.1 - 5 % w/w (+)-catechin, (-)-catechin , (+)-epicatechin, (-)-epicatechin or mixtures thereof.

5. A method of colouring hair fibres according to any one of the preceding claims, wherein the hair colour composition comprises 0.0001 - 3 preferably 0.001 - 1, most preferably 0.01 - 1 % w/w hydrogen peroxide.

6. A method of colouring hair fibres according to any one of the preceding claims, wherein the hair colour composition comprises 0.0001 - 5, preferably 0.001 - 1 % w/w peroxidase.

7. A method of colouring hair fibres according to any one of the preceding claims, wherein the hydrogen peroxide generator comprises a hydrogen peroxide generating oxidase, a substrate and oxygen.

8. A method of colouring hair fibres according to claim 9, wherein the hydrogen peroxide generating oxidase is selected from the group consisting of (S)-2-hydroxy acid oxidase, D-galactose oxidase, glucose oxidase, coniferyl alcohol oxidase, glycolate oxidase, hexose oxidase, oxalate oxidase, amino acid oxidase and L-galactonolactone oxidase and the respective substrate is selected from the group consisting of (S)-2-hydroxy acid, D-galactose, glucose, coniferyl alcohol, α-hydroxy acids, D-glucose, oxalic acid, amino acid and L-galactono-1,4-lactone.

9. A method of colouring hair fibres according to any one of the preceding claims, wherein the hydrogen peroxide generator is selected from the group consisting of (S)-2-hydroxy acid with (S)-2-hydroxy acid oxidase, D-galactose with D-galactose oxidase, glucose with glucose oxidase, coniferyl alcohol with coniferyl alcohol oxidase, α-hydroxy acids with glycolate oxidase, D-glucose with hexose oxidase, oxalic acid with oxalate oxidase, and L-galactono-1,4-lactone with L-galactonolactone oxidase, amino acid oxidase with amino acids, all in the presence of oxygen.

**10.** A method of colouring hair fibres according to any one of claims 9 to 11, wherein the hair colour composition comprises 0.0001 - 5, preferably 0.001 - 1 % w/w hydrogen peroxide generating oxidase.

**11.** A method of colouring hair fibres according to any one of claims 9 to 12, wherein the hair colour composition comprises 0.01 - 10 preferably 0.1 - 5 % w/w substrate.

**12.** A method of colouring hair fibres according to any one of the preceding claims additionally comprising a metal ion suitable for coordinating to (+)-catechin, (-)-catechin, (+)-epicatechin, (-)-epicatechin or mixtures thereof, or the product of the reaction of (+)-catechin, (-)-catechin, (+)-epicatechin, (-)-epicatechin or mixtures thereof in the presence of the peroxidase and hydrogen peroxide.

**13.** A method of colouring hair fibres according to claim 1, wherein the metal ion is selected from the group consisting of iron (II), iron (III), copper (I), copper (II), copper (III) and aluminium (III).

**14.** A method of colouring hair fibres according to claim 12 or claim 13, wherein the hair colour composition comprises 0.0001 - 2, preferably 0.001 - 0.1 % w/w metal ion.

**Patentansprüche**

**1.** Verfahren zum Färben von Haarfasern,
wobei das Verfahren den Schritt des Auftragens einer Haarfärbezusammensetzung aufweist, wobei die Haarfärbezusammensetzung Folgendes aufweist:

(a) (+)-Catechin, (-)-Catechin, (+)-Epicatechin, (-)-Epicatechin oder Gemische davon;
(b) einen Wasserstoffperoxiderzeuger oder Wasserstoffperoxid; und
(c) eine Peroxidase;

wobei die Zusammensetzung einen pH-Wert von 4,5 bis 7,0, vorzugsweise von weniger als oder gleich 6,0 aufweist.

**2.** Verfahren zum Färben von Haarfasern nach Anspruch 1,
wobei die Peroxidase eine nicht von tierischem Blut stammende Peroxidase aus der Klasse II (Pilze) oder der Klasse III (Pflanzen und Algen) ist.

**3.** Verfahren zum Färben von Haarfasern nach Anspruch 2,
wobei die Peroxidase aus der Gruppe erhalten wird, bestehend aus Arabidopsis thaliana, Meerrettich, Gerste, Erdnuss, Sojabohnen, Tabak und Rübe (Pflanzen), Chlorophyta spirogyra (Grünalge), Arthromyces ramosus und Corprinus cinereus (Pilze).

**4.** Verfahren zum Färben von Haarfasern nach einem der vorstehenden Ansprüche,
wobei die Haarfärbezusammensetzung 0,01 bis 10, vorzugsweise 0,1 bis 5 Gew.-% (+)-Catechin, (-)-Catechin , (+)-Epicatechin, (-)-Epicatechin oder Gemische davon aufweist.

**5.** Verfahren zum Färben von Haarfasern nach einem der vorstehenden Ansprüche,
wobei die Haarfärbezusammensetzung 0,0001 bis 3, vorzugsweise 0,001 bis 1, besonders bevorzugt 0,01 bis 1 Gew.-% Wasserstoffperoxid aufweist.

**6.** Verfahren zum Färben von Haarfasern nach einem der vorstehenden Ansprüche,
wobei die Haarfärbezusammensetzung 0,0001 bis 5, vorzugsweise 0,001 bis 1 Gew.-% Peroxidase aufweist.

**7.** Verfahren zum Färben von Haarfasern nach einem der vorstehenden Ansprüche,
wobei der Wasserstoffperoxiderzeuger eine Wasserstoffperoxid erzeugende Oxidase, ein Substrat und Sauerstoff aufweist.

**8.** Verfahren zum Färben von Haarfasern nach Anspruch 9,
wobei die Wasserstoffperoxid erzeugende Oxidase aus der Gruppe ausgewählt ist, bestehend aus (S)-2-Hydroxylsäureoxidase, D-Galactoseoxidase, Glucoseoxidase, Coniferylalkoholoxidase, Glycolatoxidase, Hexoseoxidase, Oxalatoxidase, Aminosäureoxidase und L-Galactonolactonoxidase, und das entsprechende Substrat aus der Gruppe ausgewählt ist, bestehend aus (S)-2-Hydroxylsäure, D-Galactose, Glucose, Coniferylalkohol, α-Hydroxylsäuren, D-Glucose, Oxalsäure, Aminosäure und L-Galactono-1,4-lacton.

**9.** Verfahren zum Färben von Haarfasern nach einem der vorstehenden Ansprüche, wobei der Wasserstoffperoxiderzeuger aus der Gruppe ausgewählt ist, bestehend aus (S)-2-Hydroxylsäure mit (S)-2-Hydroxylsäureoxidase, D-Galactose mit D-Galactoseoxidase, Glucose mit Glucoseoxidas, Coniferylalkohol mit Coniferylalkoholoxidase, α-Hydroxylsäuren mit Glycolatoxidase, D-Glucose mit Hexoseoxidase, Oxalsäure mit Oxalatoxidase und L-Galactono-1,4-lacton mit L-Galactonolactonoxidase, Aminosäureoxidase mit Aminosäuren, jeweils in Gegenwart von Sauerstoff.

**10.** Verfahren zum Färben von Haarfasern nach einem der Ansprüche 9 bis 11, wobei die Haarfärbezusammensetzung 0,0001 bis 5, vorzugsweise 0,001 bis 1 Gew.-% Wasserstoffperoxid erzeugende Oxidase aufweist.

**11.** Verfahren zum Färben von Haarfasern nach einem der Ansprüche 9 bis 12, wobei die Haarfärbezusammensetzung 0,01 bis 10, vorzugsweise mit 0,1 bis 5 Gew.-% Substrat aufweist.

**12.** Verfahren zum Färben von Haarfasern nach einem der vorstehenden Ansprüche, die ferner ein Metallion aufweist, das für die Koordination mit (+)-Catechin, (-)-Catechin, (+)-Epicatechin, (-)-Epicatechin oder Gemischen davon oder dem Reaktionsprodukt von (+)-Catechin, (-)-Catechin, (+)-Epicatechin, (-)-Epicatechin oder Gemischen davon in Gegenwart von der Peroxidase und Wasserstoffperoxid geeignet ist.

**13.** Verfahren zum Färben von Haarfasern nach Anspruch 1, wobei das Metallion aus der Gruppe ausgewählt ist, die aus Eisen(II), Eisen(III), Kupfer(I), Kupfer(II), Kupfer(III) und Aluminium(III) besteht.

**14.** Verfahren zum Färben von Haarfasern nach Anspruch 12 oder 13, wobei die Haarfärbezusammensetzung 0,0001 bis 2, vorzugsweise 0,001 bis 0,1 Gew.-% Metallion aufweist.

## Revendications

**1.** Procédé de coloration de fibres capillaires, le procédé comprenant l'étape d'application d'une composition de couleur capillaire, la composition de couleur capillaire comprenant :

(a) de la (+)-catéchine, (-)-catéchine, (+)-épicatéchine, (-)-épicatéchine ou des mélanges de celles-ci ;
(b) un générateur de peroxyde d'hydrogène ou du peroxyde d'hydrogène ; et
(c) une peroxydase et;

dans lequel la composition présente un pH de 4,5 à 7,0, de préférence inférieur ou égal à 6,0.

**2.** Procédé de coloration de fibres capillaires selon la revendication 1, dans lequel la peroxydase est une peroxydase à hème non-animale de la classe I (fungi) ou de la classe III (plantes et algues).

**3.** Procédé de coloration de fibres capillaires selon la revendication 2, dans lequel la peroxydase est obtenue dans le groupe constitué d'Arabidopsis thaliana, de raifort, d'orge, d'arachide, de soja, de tabac, et de navet (plantes), de Chlorophyta spirogyra (algues vertes), d'Arthromyces ramosus et de Corprinus cinereus (fungi).

**4.** Procédé de coloration de fibres capillaires selon l'une quelconque des revendications précédentes, dans lequel la composition de couleur capillaire comprend 0,01-10, de préférence 0,1-5 % en masse/ masse de (+)-catéchine, (-)-catéchine, (+)-épicatéchine, (-)-épicatéchine ou de mélanges de celles-ci.

**5.** Procédé de coloration de fibres capillaires selon l'une quelconque des revendications précédentes, dans lequel la composition de couleur capillaire comprend 0,0001 - 3, de préférence 0,001 - 1, encore mieux 0,01 - 1 % en masse/masse de peroxyde d'hydrogène.

**6.** Procédé de coloration de fibres capillaires selon l'une quelconque des revendications précédentes, dans lequel la composition de couleur capillaire comprend 0,0001 - 5, de préférence 0,001 - 1 % en masse/masse de peroxydase.

**7.** Procédé de coloration de fibres capillaires selon l'une quelconque des revendications précédentes, dans lequel le générateur de peroxyde d'hydrogène comprend une oxydase générant du peroxyde d'hydrogène, un substrat et de l'oxygène.

**8.** Procédé de coloration de fibres capillaires selon la revendication 9, dans lequel l'oxydase générant du peroxyde d'hydrogène est choisie dans le groupe constitué de (S)-2-hydroxyacide oxydase, D-galactose oxydase, glucose oxydase, alcool coniférylique oxydase, glycolate oxydase, hexose oxydase, oxalate oxydase, amino-acide oxydase et L-galactonolactone oxydase et le substrat respectif est choisi dans le groupe constitué de (S)-2-hydroxyacide, D-galactose, glucose, alcool coniférylique, $\alpha$-hydroxyacides, D-glucose, acide oxalique, amino-acide et L-galactono-1,4-lactone.

**9.** Procédé de coloration de fibres capillaires selon l'une quelconque des revendications précédentes, dans lequel le générateur de peroxyde d'hydrogène est choisi dans le groupe constitué de (S)-2-hydroxyacide avec de la (S)-2-hydroxyacide oxydase, D-galactose avec de la D-galactose oxydase, glucose avec de la glucose oxydase, alcool coniférylique avec de l'alcool coniférylique oxydase, $\alpha$-hydroxyacides avec de la glycolate oxydase, D-glucose avec de l'hexose oxydase, acide oxalique avec de l'oxalate oxydase, et L-galactono-1,4-lactone avec de la L-galactonolactone oxydase, amino-acide oxydase avec des amino-acides, tous en présence d'hydrogène.

**10.** Procédé de coloration de fibres capillaires selon l'une quelconque des revendications 9 à 11, dans lequel la composition de couleur capillaire comprend 0,0001 - 5, de préférence 0,001 - 1 % en masse/masse d'oxydase générant du peroxyde d'hydrogène.

**11.** Procédé de coloration de fibres capillaires selon l'une quelconque des revendications 9 à 12, dans lequel la composition de couleur capillaire comprend 0,01 - 10, de préférence 0,1 - 5 % en masse/ masse de substrat.

**12.** Procédé de coloration de fibres capillaires selon l'une quelconque des revendications précédentes comprenant de plus un ion métallique approprié pour se coordonner à la (+)-catéchine, (-)-catéchine, (+)-épicatéchine, (-)-épicatéchine ou des mélanges de celles-ci, ou le produit de la réaction de (+)-catéchine, (-)-catéchine, (+)-épicatéchine, (-)-épicatéchine ou des mélanges de ceux-ci en présence de la peroxydase et du peroxyde d'hydrogène.

**13.** Procédé de coloration de fibres capillaires selon la revendication 1, dans lequel l'ion métallique est choisi dans le groupe constitué de fer (II), fer (III), cuivre (I), cuivre (II), cuivre (III) et aluminium (III).

**14.** Procédé de coloration de fibres capillaires selon la revendication 12 ou la revendication 13, dans lequel la composition de couleur capillaire comprend 0,0001 - 2, de préférence 0,001 - 0,1 % en masse/masse d'ion métallique.

Fig. 1a

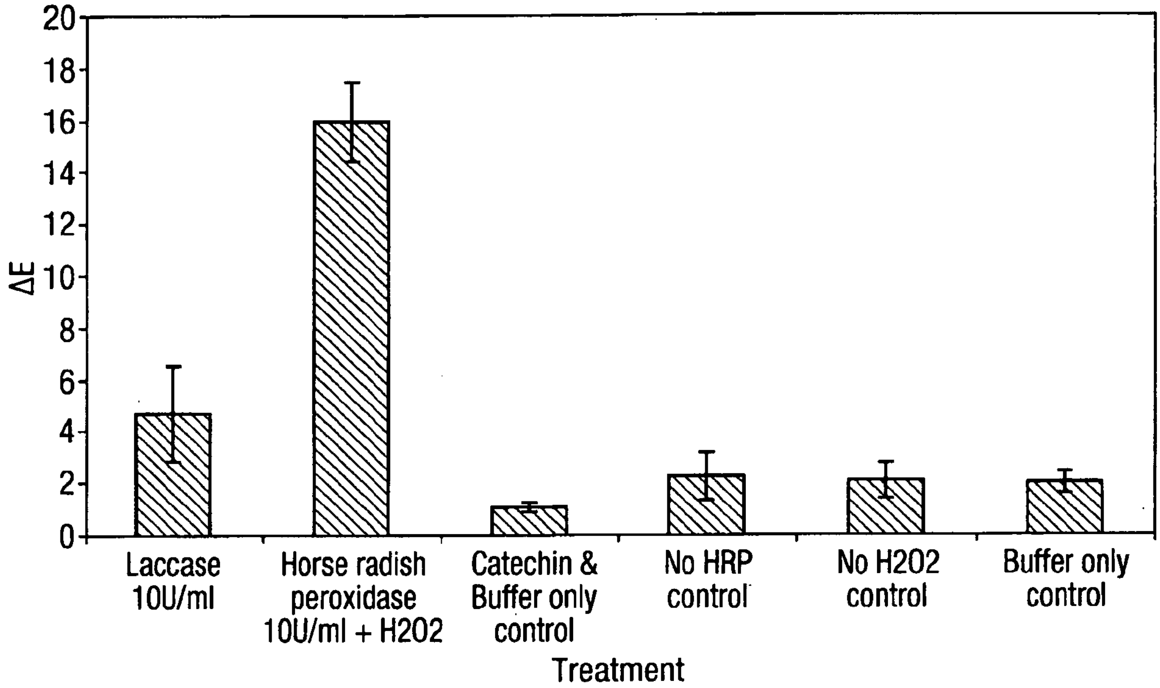
ΔE
20
18
16
14
12
10
8
6
4
2
0
Laccase 10U/ml
Horse radish peroxidase 10U/ml + H2O2
Catechin & Buffer only control
No HRP control
No H2O2 control
Buffer only control
Treatment

Fig. 1b

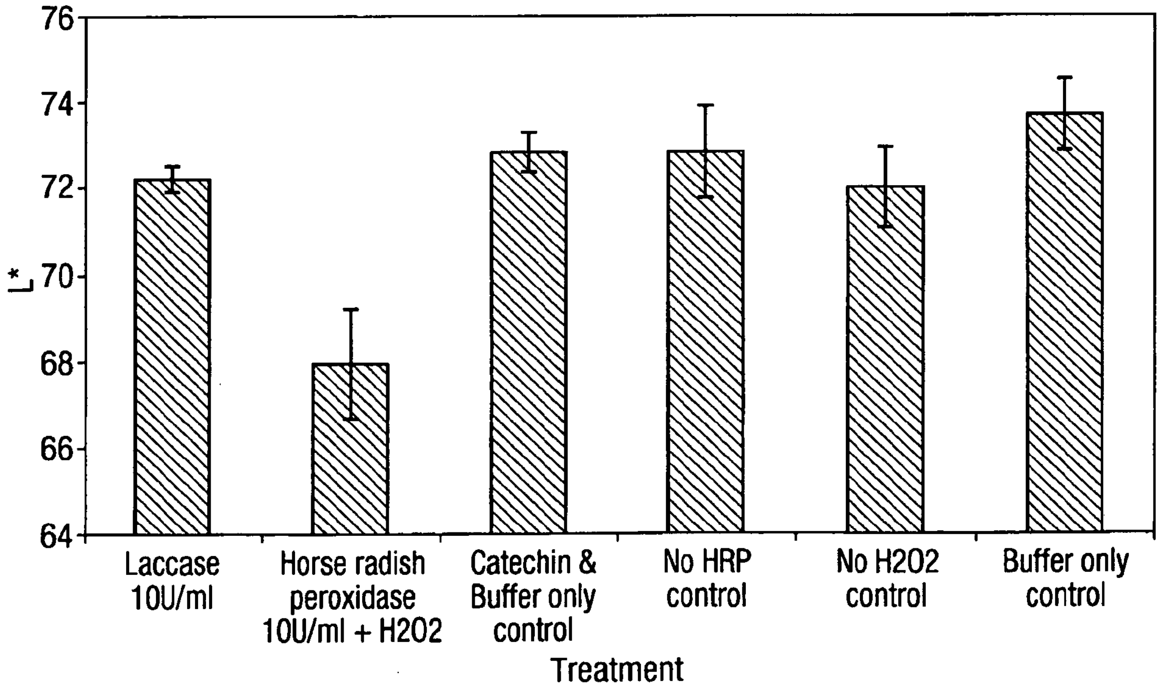
L*
76
74
72
70
68
66
64
Laccase 10U/ml
Horse radish peroxidase 10U/ml + H2O2
Catechin & Buffer only control
No HRP control
No H2O2 control
Buffer only control
Treatment

Fig. 1c

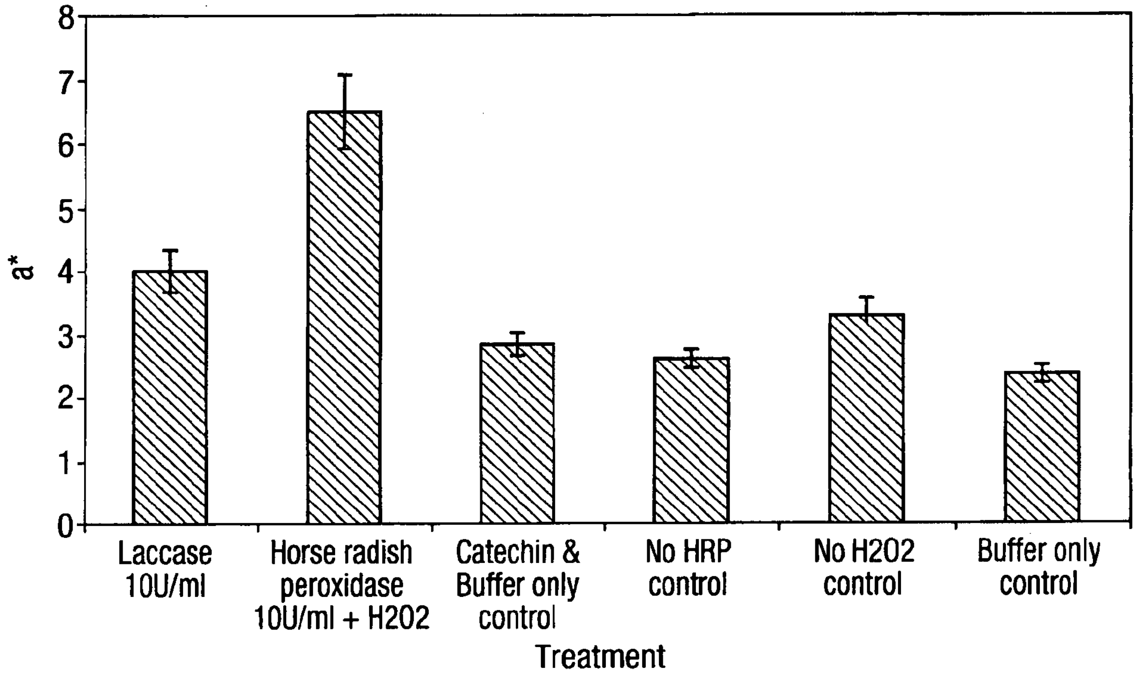
8
7
6
5
4
3
2
1
0
a*
Laccase 10U/ml
Horse radish peroxidase 10U/ml + H2O2
Catechin & Buffer only control
No HRP control
No H2O2 control
Buffer only control
Treatment

Fig. 1d

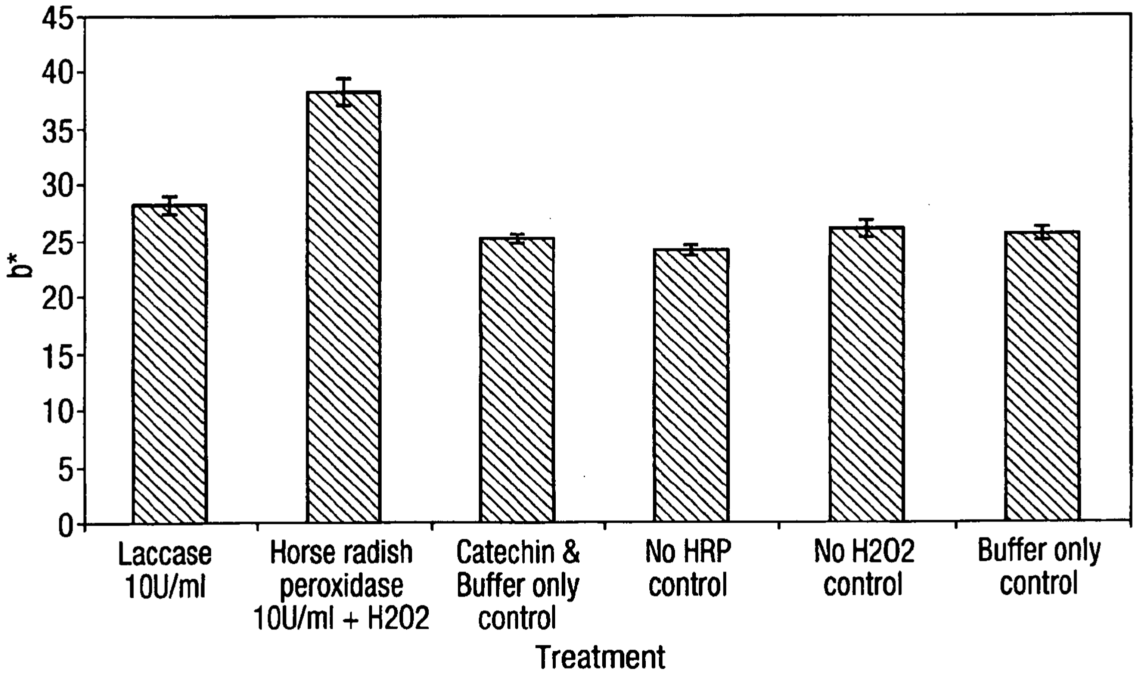
45
40
35
30
25
20
15
10
5
0
b*
Laccase 10U/ml
Horse radish peroxidase 10U/ml + H2O2
Catechin & Buffer only control
No HRP control
No H2O2 control
Buffer only control
Treatment

# Fig. 2a

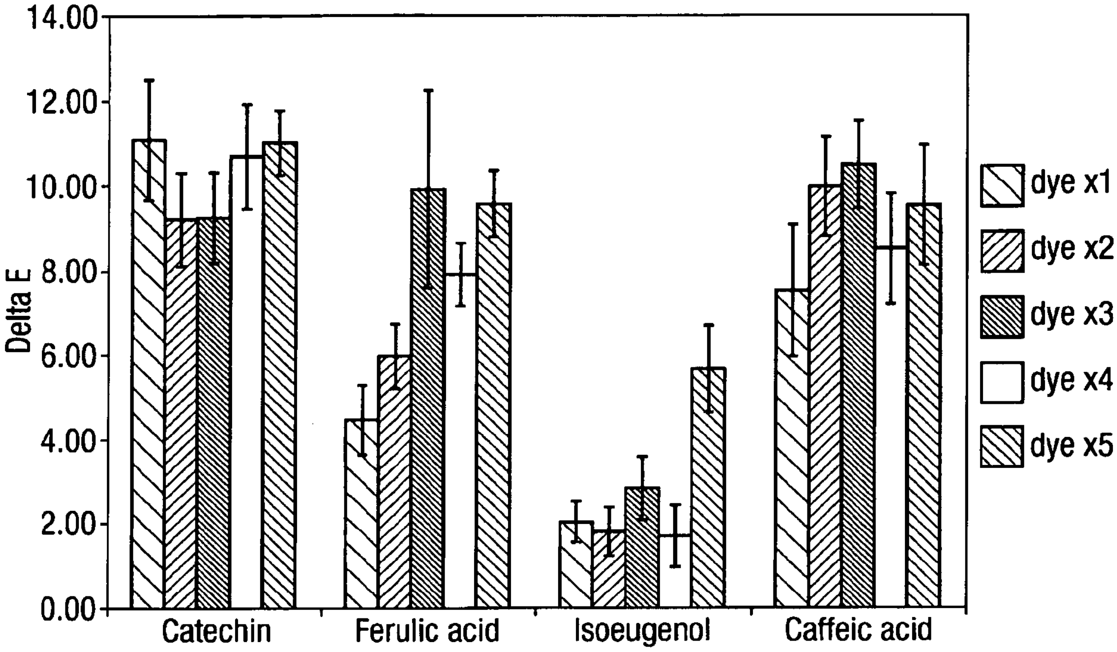
14.00
12.00
10.00
8.00
6.00
4.00
2.00
0.00
Delta E
Catechin
Ferulic acid
Isoeugenol
Caffeic acid
dye x1
dye x2
dye x3
dye x4
dye x5

# Fig. 2b

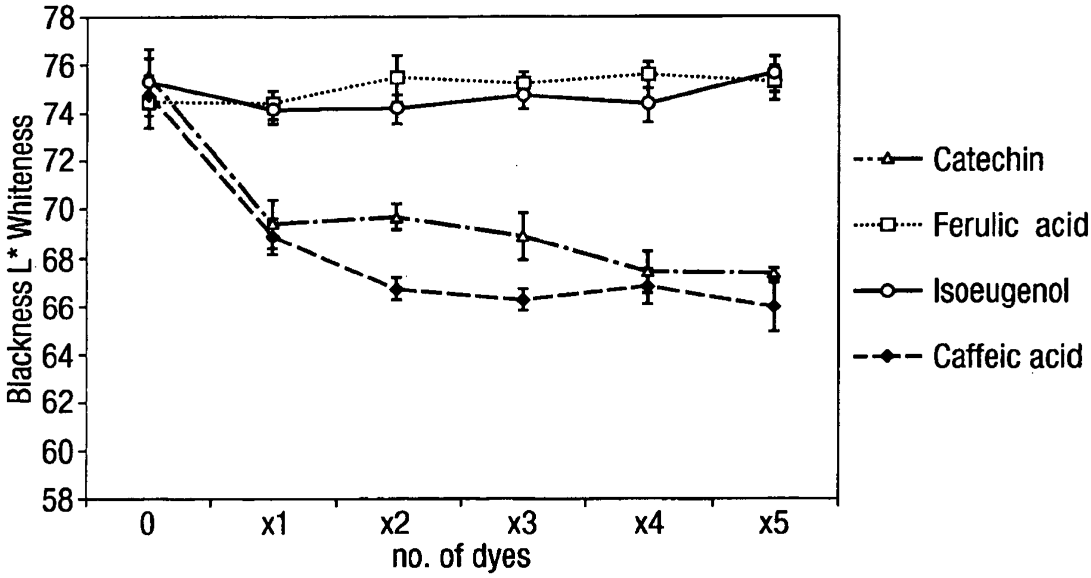
78
76
74
72
70
68
66
64
62
60
58
Blackness L* Whiteness
0
x1
x2
x3
x4
x5
no. of dyes
Catechin
Ferulic acid
Isoeugenol
Caffeic acid

Fig. 2c

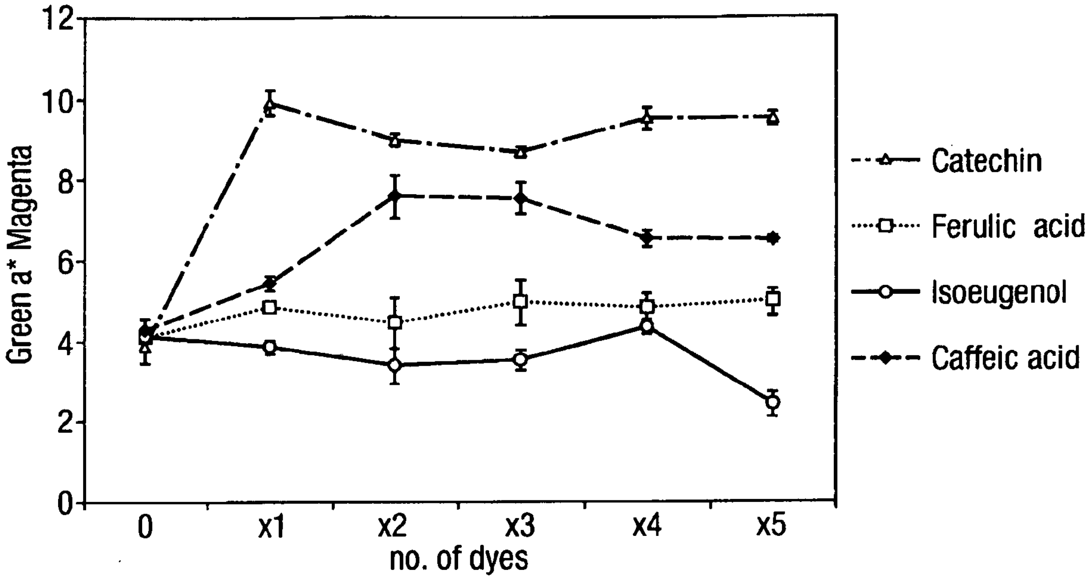
12
10
8
6
4
2
0
Green a* Magenta
0
x1
x2
x3
x4
x5
no. of dyes
Catechin
Ferulic acid
Isoeugenol
Caffeic acid

Fig. 2d

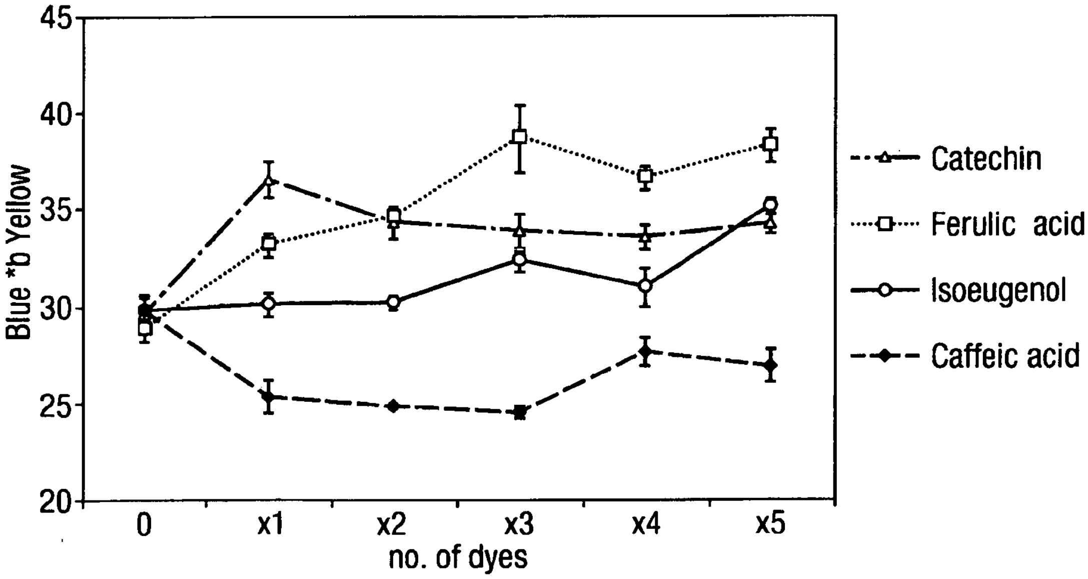
45
40
35
30
25
20
Blue *b Yellow
0
x1
x2
x3
x4
x5
no. of dyes
Catechin
Ferulic acid
Isoeugenol
Caffeic acid

Fig. 3a

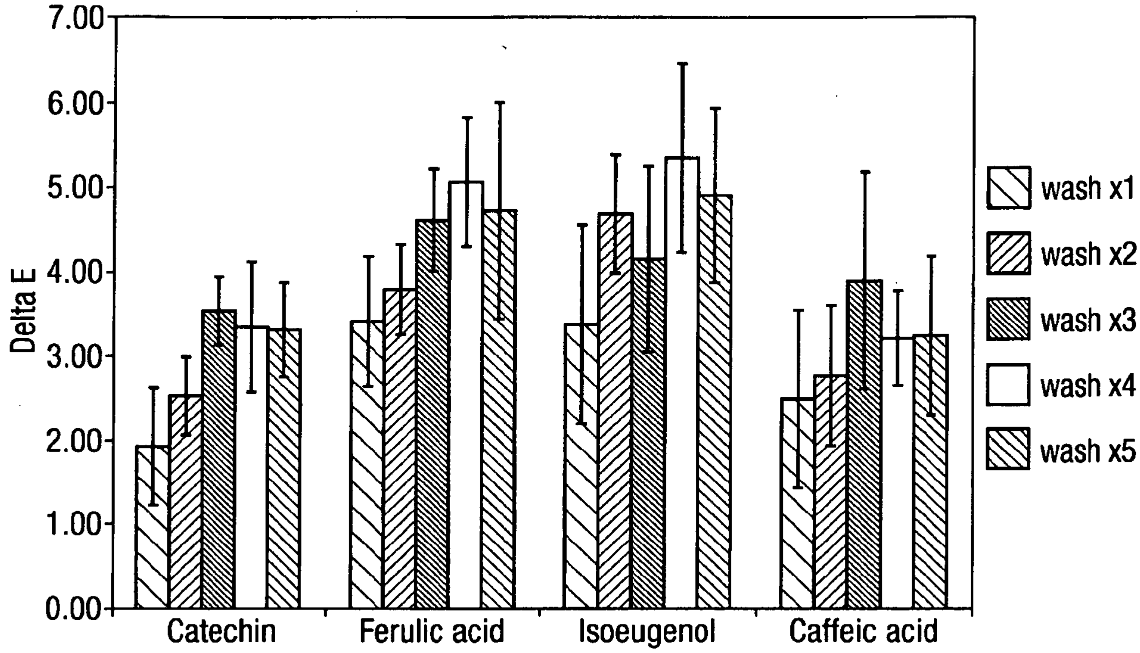
7.00
6.00
5.00
4.00
3.00
2.00
1.00
0.00
Delta E
Catechin
Ferulic acid
Isoeugenol
Caffeic acid
wash x1
wash x2
wash x3
wash x4
wash x5

Fig. 3b

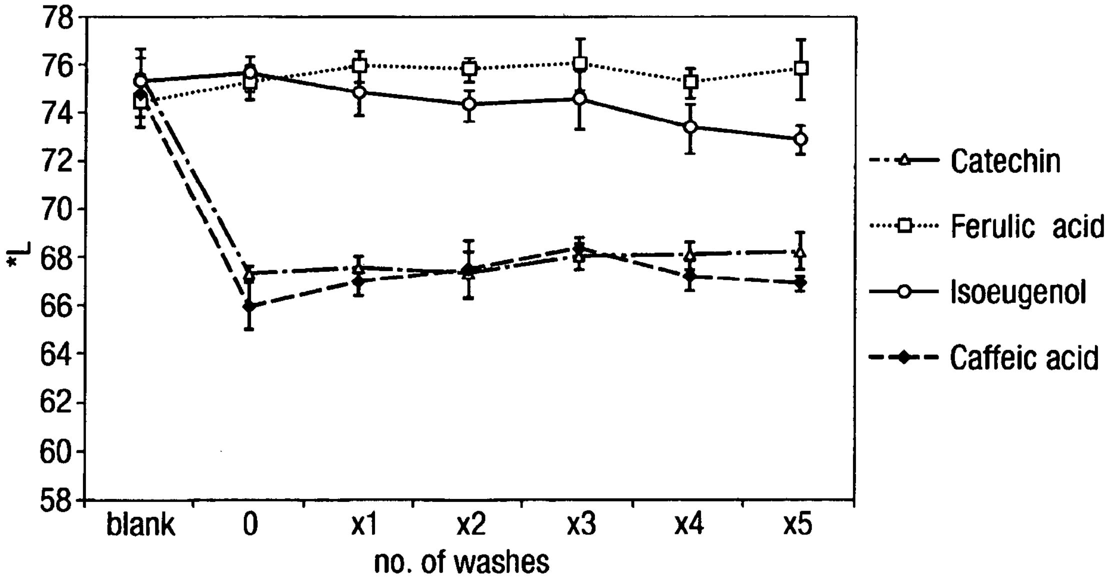
78
76
74
72
70
68
66
64
62
60
58
*L
blank
0
x1
x2
x3
x4
x5
no. of washes
Catechin
Ferulic acid
Isoeugenol
Caffeic acid

Fig. 3c

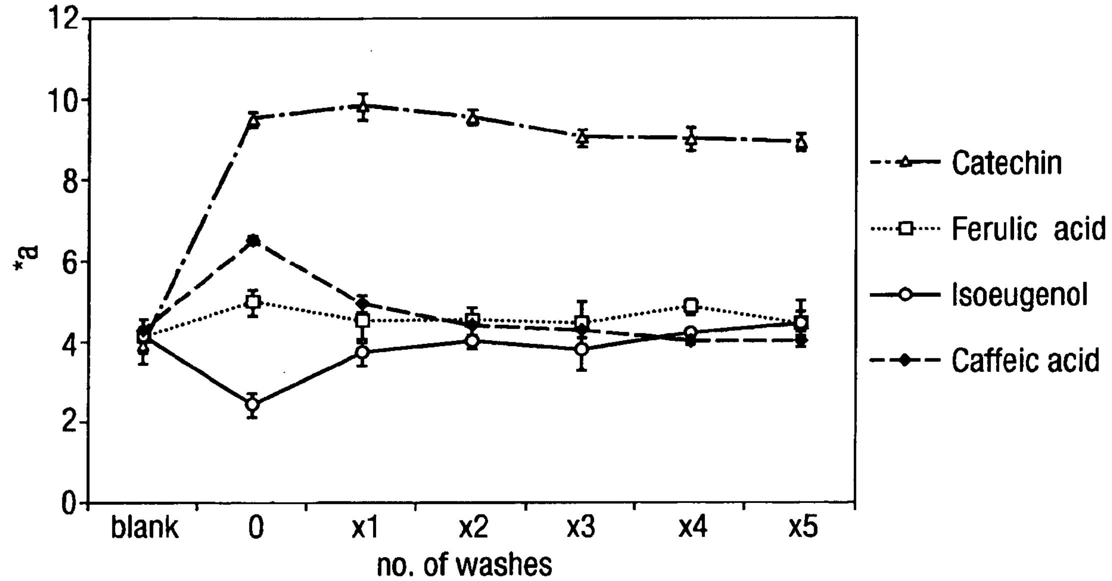
12
10
8
6
4
2
0
*a
blank
0
x1
x2
x3
x4
x5
no. of washes
Catechin
Ferulic acid
Isoeugenol
Caffeic acid

Fig. 3d

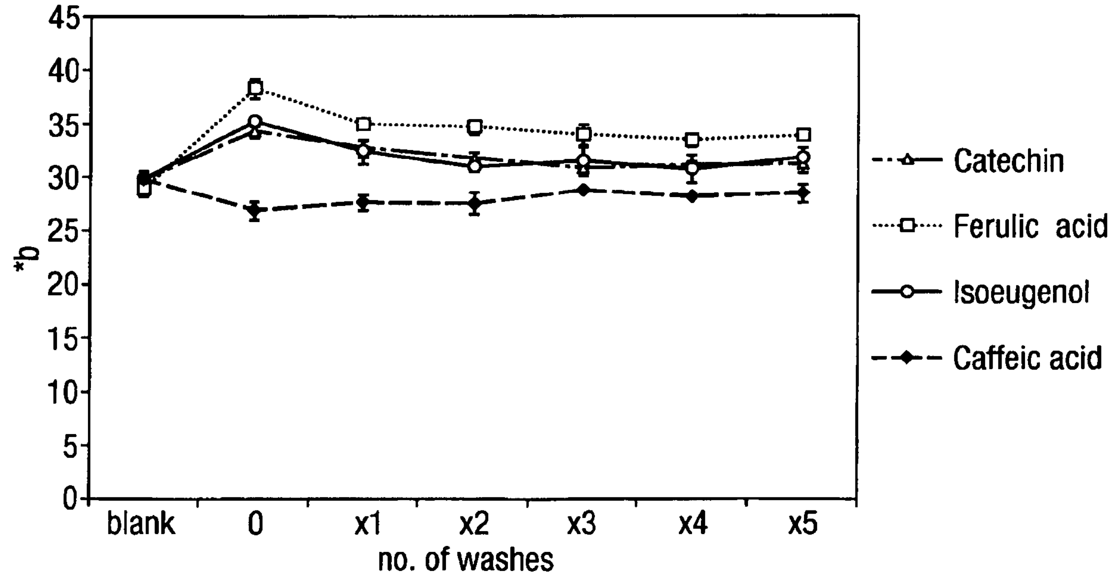
45
40
35
30
25
20
15
10
5
0
*b
blank
0
x1
x2
x3
x4
x5
no. of washes
Catechin
Ferulic acid
Isoeugenol
Caffeic acid

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6572843 B
- US 3957424 A


### Non-patent literature cited in the description


- **TAKADA et al.** *Journal of Oleo Science,* 2003, vol. 52 (10), 557-563
- **T. VUORINEN.** *Carbohydrate Research,* 15 April 1984, vol. 127 (2), 319-325